# EUROPEAN PATENT APPLICATION

(11) **EP 4 368 235 A1**
(43) Date of publication of application: **15.05.2024**
(21) Application number: 22837067.2
(22) Date of filing: 09.07.2022
(51) Int. Cl.: A61M 39/24

(54) **ADJUSTABLE HEMOSTASIS VALVE DEVICE**

(30) Priority: 09.07.2021 CN 202110780973; 09.07.2021 CN 202121570585 U
(71) Applicant: Jiangsu Polylive Medtech Co., Ltd., Nantong, Jiangsu 226400 (CN)
(72) Inventor: ZUO, Bin, Nantong, Jiangsu 226400 (CN)
(74) Representative: Zaboliene, Reda
(86) International application number: PCT/CN2022/104767
(87) International publication number: WO 2023/280322

(57) **Abstract**

An adjustable hemostasis valve device (100), comprising a catheter sheath housing (1), a catheter sheath cap (2), a sealing member (3) and an opening adjustment mechanism (12), wherein the catheter sheath housing (1) is connected to the catheter sheath cap (2), the catheter sheath housing (1) and the catheter sheath cap (2) can axially move relative to each other, and the sealing member (3) and the opening adjustment mechanism (12) are connected to each other and then arranged in the catheter sheath housing (1); the opening adjustment mechanism (12) comprises a first positioning plate (4) and a mounting cylinder (5), and the sealing member (3) is fixedly connected to the first positioning plate (4) and then mounted in the mounting cylinder (5); the sealing member (3) comprises an axially extending spiral flow channel (301); the sealing member (3) has a distal end fixed to the mounting cylinder (5) and a proximal end fixedly connected to the first positioning plate (4), a plurality of first positioning pins (401) are distributed on the periphery of the first positioning plate (4), and a plurality of spiral sliding guide grooves (501) are provided in a proximal end of the mounting cylinder (5); and the axial movement of the catheter sheath cap (2) enables the first positioning pins (401) of the first positioning plate (4) to slide along the sliding guide grooves (501) of the mounting cylinder (5), such that the sealing member (3) is compressed and rotated or is released and rotated, so as to adjust the opening size of the spiral flow channel (301) of the sealing member (3).

## Description

The present application claims priority of Chinese Patent Application No. 202110780973.1 and entitled "ADJUSTABLE HEMOSTASIS VALVE DEVICE", filed on July 09, 2021, and Chinese Patent Application No. 202121570585.2 and entitled "ADJUSTABLE HEMOSTASIS VALVE DEVICE", filed on July 09, 2021, the entire content of which is incorporated herein by reference.

### TECHNICAL FIELD

The present invention relates to the field of medical devices, in particular to an adjustable hemostasis valve device.

### BACKGROUND TECHNOLOGY

Interventional therapy is a minimally invasive procedure that utilizes guidance of medical imaging equipment to introduce precision instruments, such as special guide wires, catheter sheaths, and the like, into human bodies to diagnose and locally treat pathologies within the bodies. It can treat illnesses that could not be treated in the past or that have not good treatment effect, while also has advantages of less trauma, quicker recovery, and better effect, and is one of directions of medical development in the future.

During an interventional procedure, one or more catheter sheaths are generally inserted through a vascular puncture technique into a blood vessel or tissue lumen and extended to the vicinity of the tissue or structure targeted by the procedure, thereby establishing a channel from outside the body to the lumen of the surgical tissue in the body to deliver minimally invasive surgical instruments and medications for the procedure or achieve real-time monitoring of medical images in the body. In order to prevent air from entering the blood vessel via the catheter sheath, reduce blood loss during the procedure, and ensure the sterility of the blood vessel during the procedure, catheter tip ends of the catheter sheaths used in the interventional procedure must be equipped with hemostasis valves that have a certain degree of sealing. Firstly, when the instrument is inserted into or withdrawn from a sheath, it needs to be sealed effectively; and secondly, the frictional resistance of the hemostasis valve to the instrument entering or leaving a catheter sheath should be as small as possible, otherwise it will affect a doctor's control to the instrument during a procedure.

### SUMMARY OF THE DISCLOSURE

### Technical Problem

Traditional hemostasis valves are generally processed from elastic materials such as silica gel, silicone rubber, etc., with pre-cut I-shape, cross or round openings in the middle of valve bodies, their sealing is realized by squeezing the silica gel elastomer, and the sizes of the openings determine the sizes of instruments that can be passed. When a surgery needs to pass instruments and catheters in large sizes, on the one hand, the deformation of the silica gel is too large, there is the possibility of failure, and at the same time, the friction with the instruments increases dramatically, which affects a doctor's control to the instruments and increases difficulty and risk of the surgery. On the other hand, the silica gel elastomer is difficult to reset after the instruments are withdrawn, and the channel loses sealness.

### Technical Solution

A purpose of the present invention is to provide an adjustable hemostasis valve device, which is capable of adjusting the size of an opening of a sealing member in the adjustable hemostasis valve device to accommodate passage of surgical instruments of different standards and to maintain good sealing performance and pushing performance during their passage.

The present invention provides an adjustable hemostasis valve device comprising a catheter sheath and a sealing member, the catheter sheath is internally hollow, the sealing member is located within the catheter sheath, the sealing member is an elastic member comprising a helical flow channel extending axially, a proximal end and a distal end of the catheter sheath is movable relative to each other axially, and the proximal end of the catheter sheath, when moving axially, is capable of compressing and rotating, or releasing and rotating, the sealing member to adjust the size of an opening of the helical flow channel of the sealing member to accommodate different standards of surgical instruments passing through the helical flow channel.

Furthermore, the catheter sheath comprises a catheter sheath housing located at the distal end and a catheter sheath cap located at the proximal end, the catheter sheath housing and the catheter sheath cap are movable relative to each other axially; a distal end portion of the catheter sheath cap extends inwardly to form a limit ring, a position of the catheter sheath housing close to the proximal end is provided annularly with a limit bump, and the limit ring of the catheter sheath cap and the limit bump of the catheter sheath housing cooperate with each other to limit the relative displacement stroke between the catheter sheath cap and the catheter sheath housing.

Furthermore, an opening adjustment mechanism is further included, the opening adjustment mechanism comprises a first positioning disk and a mounting cylinder, the first positioning disk is fixedly connected to the proximal end of the sealing member, the sealing member is mounted in the mounting cylinder after being fixedly connected to the first positioning disk, a plurality of first positioning pins are distributed around the outer periphery of the first positioning disk, and a plurality of helical sliding guide grooves matching the positioning pins are formed at the proximal end of the mounting cylinder; when the catheter sheath cap moves axially, the first positioning pins of the first positioning disk slide along the sliding guide grooves of the mounting cylinder to adjust the size of the opening of the helical flow channel of the sealing member.

Furthermore, the opening adjustment mechanism further comprises a second positioning disk, the second positioning disk is fixedly connected to the distal end of the sealing member, a plurality of second positioning pins are distributed around the outer periphery of the second positioning disk, and a plurality of positioning holes are formed at the distal end of the mounting cylinder; the second positioning pins of the second positioning disk cooperate with the positioning holes of the mounting cylinder to fix the distal end of the sealing member with the mounting cylinder.

Furthermore, surfaces of both the first positioning disk and the second positioning disk close to the sealing member are provided with first steps, corresponding positions of the two ends of the sealing member are provided with second steps, the first steps and the second steps are staggered along a circumferential direction, and the first positioning disk and the second positioning disk are bonded together with the sealing member through the first steps and the second steps.

Furthermore, a one-way valve is further included, the one-way valve is provided axially at two ends or in the middle of the sealing member.

Furthermore, a push ring is formed on the inner side of the catheter sheath cap, a distal end of the push ring is in contact with a proximal end of the first positioning disk, and the push ring is coupled with the first positioning disk when the catheter sheath cap moves axially.

Furthermore, a fixing ring is further included, the fixing ring is fixedly connected to the proximal end of the mounting cylinder, and an outer periphery of the fixing ring is connected by thread with the inner wall of the proximal end of the catheter sheath housing to fix the mounting cylinder within the catheter sheath housing.

Furthermore, the catheter sheath cap is connected by thread with the proximal end of the catheter sheath housing, and by rotating the catheter sheath cap, it is capable of being controlled to move along an axial direction of the catheter sheath housing.

Furthermore, a spring and a drive member are further included, the spring is sheathed on the catheter sheath housing and located between the limit ring of the catheter sheath cap and the limit bump of the catheter sheath housing, and the catheter sheath housing and the catheter sheath cap are in gap-fit; the drive member is rotatably fixed to the catheter sheath housing, a distal end of the drive member has a cam structure, the cam structure is in contact with the distal end of the catheter sheath cap for controlling displacement of the catheter sheath cap, and the catheter sheath cap is movable axially along the catheter sheath housing under the cooperative action of the spring and the cam structure.

Furthermore, the drive member comprises a pressing portion and the cam structure formed by extension from two sides of the pressing portion, the cam structure is provided thereon with a mounting shaft, the drive member is rotatably secured on the catheter sheath housing through the mounting shaft.

### Advantageous Effect

The adjustable hemostasis valve device of the present invention has advantageous effects comprising:
(1) the adjustable hemostasis valve device comprises the sealing member made of silica gel or silicone rubber, which comprises the helical flow channel located in the middle of the sealing member axially and straight-through flow channels located at the two ends of the helical flow channel, and a function of adjusting the opening size of the sealing member is achieved by compressing and rotating the helical flow channel in the center of the sealing member;
(2) the sealing member is provided with the one-way valve in a shape of three or more flap-leaves to further enhance its sealness, so that it can be used in the case that a pressure difference between distal and proximal ends thereof is large;
(3) the first positioning disk, the second positioning disk, and the mounting cylinder form the opening adjustment mechanism, the first positioning disk and the second positioning disk are provided with the first positioning pins and the second positioning pins, the mounting cylinder is provided with the helical sliding guide groove, the compressive and rotary composite motion of the sealing member is realized by axially pushing and pulling the first positioning disk, thereby adjusting the opening size of the sealing member in the adjustable hemostasis valve device;
(4) the movable catheter sheath cap with a thread structure can accurately control the axial movement of the catheter sheath cap by thread rotation so as to push the first positioning disk, thereby realizing accurate control of the opening size of the sealing member in the adjustable hemostasis valve device;
(5) the axial movement of the catheter sheath cap along the catheter sheath housing is realized through the cooperation of the spring and the cam structure of the drive member, the opening size of the sealing member in the adjustable hemostasis valve device is controlled through the drive member for passage of instruments, the spring automatically resets after the drive member is released to drive the catheter sheath cap to move axially to maintain sealness, and the drive member and the spring cooperate with each other so as to realize adaptive adjustment for instruments of different diameters.

### BRIEF DESCRIPTION OF THE DRAWINGS

In order to explain technical solutions of embodiments of the present invention more clearly, drawings required to be used in the embodiments will be briefly introduced below. It is obvious that the following drawings only show some embodiments of the present invention and thus should not be regarded as limit of the scope. For those skilled in the art, other drawings can be further obtained according to these drawings on the premise of paying no creative work.
FIG. 1 is an exploded schematic view of an adjustable hemostasis valve device in an embodiment 1.
FIG. 2 is a cut-away view of the adjustable hemostasis valve device in the embodiment 1.
FIG. 3 is a structural schematic view of a sealing member in a not compressed and not rotated state.
FIG. 4 is a cut-away view of the sealing member in the not compressed and not rotated state.
FIG. 5 is a structural schematic view of the sealing member in a compressed and rotated state.
FIG. 6 is a cut-away view of the sealing member in the compressed and rotated state.
FIG. 7 is a structural schematic view of assembly of the sealing member, a first positioning disk, and a second positioning disk.
FIG. 8 is a structural schematic view of a mounting cylinder.
FIG. 9 is a schematic view of assembly of the sealing member and a one-way valve.
FIG. 10 is an exploded schematic view of an adjustable hemostasis valve device in an embodiment 2.
FIG. 11 is a structural schematic view of the adjustable hemostasis valve device in the embodiment 2.
FIG. 12 is a cut-away view of the adjustable hemostasis valve device in the embodiment 2.
FIG. 13 is a structural schematic view of a drive member in the embodiment 2.

In the drawings: 1-catheter sheath housing; 2-catheter sheath cap; 3-sealing member; 4-first positioning disk; 5-mounting cylinder; 6-second positioning disk; 7-fixing ring; 8-one-way valve; 9-sealing ring; 10-spring; 11-drive member; 12-opening adjustment mechanism; 100-adjustable hemostasis valve device; 101-limit bump; 102-circular hole; 103-first cavity; 201-pushing ring; 202-limit ring; 203-first thread; 204-opening; 301-helical flow channel; 302-straight-through flow channel; 303-second step; 401-first positioning pin; 402-first step; 501-sliding guide groove; 502-positioning hole; 503-second cavity; 601-second positioning pin; 701-second thread; 1101-cam structure; 1102-pressing portion; 1103-connecting rod; 1104-mounting shaft.

### DETAILED DESCRIPTION

Specific embodiments of the present invention will be described clearly below in conjuction with the drawings. Obviously, the described embodiments are merely some embodiments of the present invention, rather than all embodiments. Based on the description of the present invention, any other embodiment obtained by one of ordinary skill in the art on the premise of paying no creative work belongs to the protection scope of the present invention.

### First Embodiment

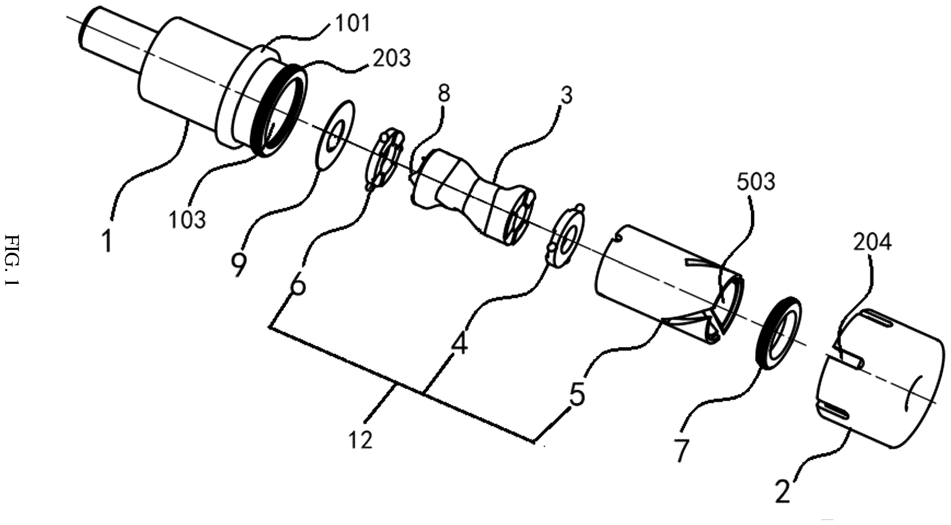

Referring to FIG. 1-FIG. 2, an adjustable hemostasis valve device 100 includes a catheter sheath, a sealing member 3, and an opening adjustment mechanism 12; the catheter sheath is internally hollow and includes a catheter sheath housing 1 located at a distal end thereof and a catheter sheath cap 2 located at a proximal end thereof, each of the catheter sheath housing 1 and the catheter sheath cap 2 is provided with a channel capable of allowing surgical instruments to enter; the catheter sheath housing 1 has a first cavity 103 therein, the sealing member 3 is connected with the opening adjustment mechanism 12 and then fixed in the first cavity 103 of the catheter sheath housing 1; both an inner wall of the catheter sheath cap 2 and an outer wall of the proximal end of the catheter sheath housing 1 are provided annularly with first thread 203, the catheter sheath cap 2 is connected by thread to the proximal end of the catheter sheath housing 1 through the first thread 203, and rotating the catheter sheath cap 2 can control it to move along an axial direction of the catheter sheath housing 1.

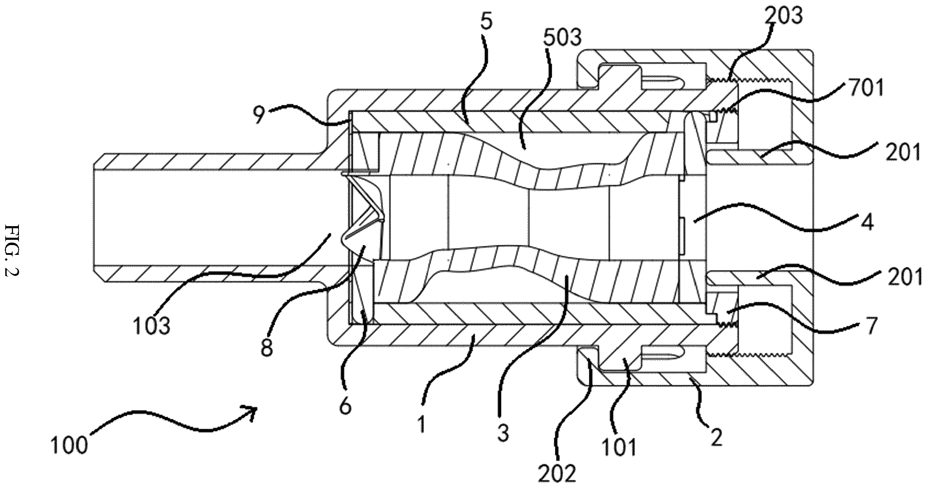

Referring to FIG. 2-FIG. 6, the sealing member 3 is an elastic member made of silica gel or silicone rubber molded in one piece, which includes a helical flow channel 301 located axially in the middle of the sealing member 3 and straight-through flow channels 302 located at the two ends of the helical flow channel 301, and a cross-section of the helical flow channel 301 may be triangular or polygonal spiral.

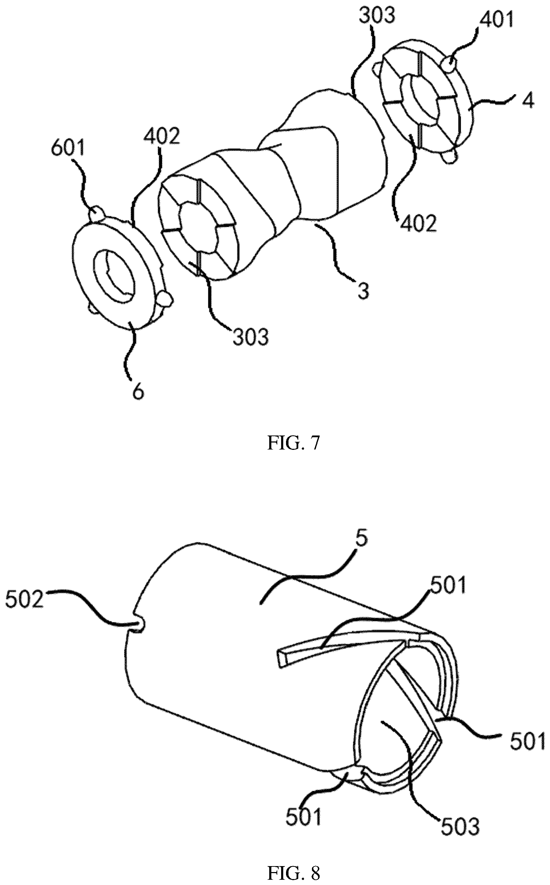

Referring to FIG. 2, FIG. 7, and FIG. 8, the opening adjustment mechanism 12 includes a first positioning disk 4, a second positioning disk 6, and a mounting cylinder 5; the mounting cylinder 5 has a second cavity 503 therein, the sealing member 3 is fixed by adhesive bonding or melting with the first positioning disk 4 and the second positioning disk 6 and then mounted in the second cavity 503 of the mounting cylinder 5. Among them, as shown in FIG. 2 and FIG. 7, the proximal end of the sealing member 3 is fixedly connected to the first positioning disk 4, and the distal end of the sealing member 3 is fixedly connected to the second positioning disk 6. Surfaces of both the first positioning disk 4 and the second positioning disk 6 close to the sealing member 3 are provided with first steps 402 (or patterns), corresponding positions of the two ends of the sealing member 3 are provided with second steps 303 (or patterns), the first steps 402 and the second steps 303 are staggered along a circumferential direction, and the first positioning disk 4 and the second positioning disk 6 are bonded together with the sealing member 3 through the first steps 402 and the second steps 303, thereby increasing connection strength among the first positioning disk 4, the second positioning disk 6, and the sealing member 3. More specifically, please continue to refer to FIG. 2, FIG. 7, and FIG. 8, a plurality of first positioning pins 401 are evenly distributed around the outer periphery of the first positioning disk 4, and a plurality of helical sliding guide grooves 501 configured for mounting the first positioning disk 4 are formed at the proximal end of the mounting cylinder 5, the first positioning disk 4 can slide along the guide grooves 501, and control of displacement and rotation of the first positioning disk 4 is realized by cooperation between the first positioning pins 401 and the sliding guide grooves 501. A plurality of second positioning pins 601 are evenly distributed around the outer periphery of the second positioning disk 6, and a plurality of positioning holes 502 are formed at the distal end of the mounting cylinder 5; the second positioning pins 601 of the second positioning disk 6 cooperate with the positioning holes 502 of the mounting cylinder 5 to fix the distal end of the sealing member 3 with the mounting cylinder 5. In this embodiment, the first positioning pins 401, the second positioning pins 601, the positioning holes 502, and the sliding guide grooves 501 are all equipped with three.

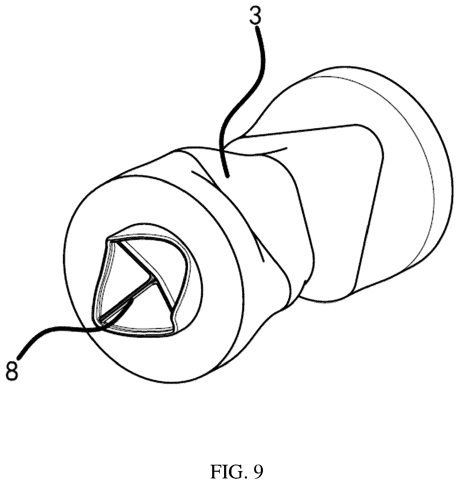

Please continue to refer to FIG. 2, FIG. 7, and FIG. 8, a push ring 201 is formed on the inner side of the catheter sheath cap 2, a distal end of the push ring 201 is in contact with a proximal end of the first positioning disk 4; by rotating the catheter sheath cap 2, it is controlled to move along the axial direction of the catheter sheath housing 1, and the push ring 201 is coupled with the first positioning disk 4 when the catheter sheath cap 2 moves axially, such that the first positioning pins 401 of the first positioning disk 4 slide along the sliding guide grooves 501 of the mounting cylinder 5, press the sealing member 3, and make the proximal end of the sealing member 3 rotate along with the first positioning disk 4, so as to realize compressive and rotary composite motion of the sealing member 3 and thus adjust the size of the opening of the helical flow channel 301 in the middle of the sealing member 3, thereby fitting passage of surgical instruments in different standards through the helical flow channel 301. More specifically, as shown in FIG. 2 and FIGS. 5-8, when the catheter sheath cap 2 is rotated clockwise, the catheter sheath cap 2 goes ahead along the axial direction of the catheter sheath housing 1, the pushing ring 201 on the catheter sheath cap 2 pushes the first positioning disk 4, the first positioning pins 401 on the first positioning disk 4 slide helically toward the distal end along the sliding guide grooves 501 on the mounting cylinder 5, rotate, and press the sealing member 3, thereby realizing the function of reducing the size of the opening of the sealing member 3 in the adjustable hemostasis valve device 100; as shown in FIGS. 2-4, FIG. 7, and FIG. 8, when the catheter sheath cap 2 is rotated counterclockwise, the catheter sheath cap 2 retreats along the axial direction of the catheter sheath housing 1, the pushing ring 201 on the catheter sheath cap 2 disengages from the first positioning disk 4, the own elasticity of the sealing member 3 pushes the first positioning disk 4 to move towards the proximal end, the first positioning pins 401 on the first positioning disk 4 slide helically toward the proximal end along the sliding guide grooves 501 on the mounting cylinder 5 until contacting with the pushing ring 201 on the catheter sheath cap 2, rotate, and release the sealing member 3, thereby realizing the function of enlarging the size of the opening of the sealing member 3 in the adjustable hemostasis valve device 100. Furthermore, referring to FIG. 1 and FIG. 9, the adjustable hemostasis valve device 100 further includes a one-way valve 8, the one-way valve 8 is axially provided on two ends or the middle of the sealing member 3, and the structure of the one-way valve 8 is a structure of three or more flap-leaves; when a pressure difference between two ends of the sealing member 3 is big, for example, in the case that the proximal end has a negative pressure, sealness of the sealing member 3 can be further enhanced. In this embodiment, the one-way valve 8 is provided at the distal end of the sealing member 3, and the flap-leaves of the one-way valve 8 are in a closed state by default; when a catheter needs to be inserted from the proximal end, the flap-leaves of the one-way valve 8 are opened to ensure passage of the catheter; when the catheter is pulled out, the shapes of the flap-leaves of the one-way valve 8 restore a closed state to maintain better sealness.

Referring to FIG. 2, an inner wall of a distal end portion of the catheter sheath cap 2 forms a limit ring 202 inwardly, a position of the catheter sheath housing 1 close to the proximal end is provided annularly with a limit bump 101, and the limit ring 202 of the catheter sheath cap 2 and the limit bump 101 of the catheter sheath housing 1 cooperate with each other to limit the relative displacement stroke between the catheter sheath cap 2 and the catheter sheath housing 1. Referring to FIG. 1, the distal end portion of the catheter sheath cap 2 is provided with a plurality of openings 204 extending axially to increase elasticity of the catheter sheath cap 2 and facilitate mounting thereof.

Referring to FIG. 1 and FIG. 2, the adjustable hemostasis valve device 100 further includes a fixing ring 7, the fixing ring 7 is fixedly connected to the proximal end of the mounting cylinder 5, both an outer circumference of the fixing ring 7 and an inner wall of the proximal end of the catheter sheath housing 1 are provided with second thread 701, the fixing ring 7 is connected by thread with the inner wall of the catheter sheath housing 1 through the second thread 701, so as to fix the mounting cylinder 5 of the opening adjustment mechanism 12 in the first cavity 103 of the catheter sheath housing 1.

Referring to FIG. 1 and FIG. 2, the adjustable hemostasis valve device 100 further includes a sealing ring 9, the sealing ring 9 is provided at a joint between distal ends of the first cavity 103 of the catheter sheath housing 1 and the second cavity 503 of the mounting cylinder 5 to ensure sealness after the first cavity 103 of the catheter sheath housing 1 and the second cavity 503 of the mounting cylinder 5 are jointed.

### Second Embodiment

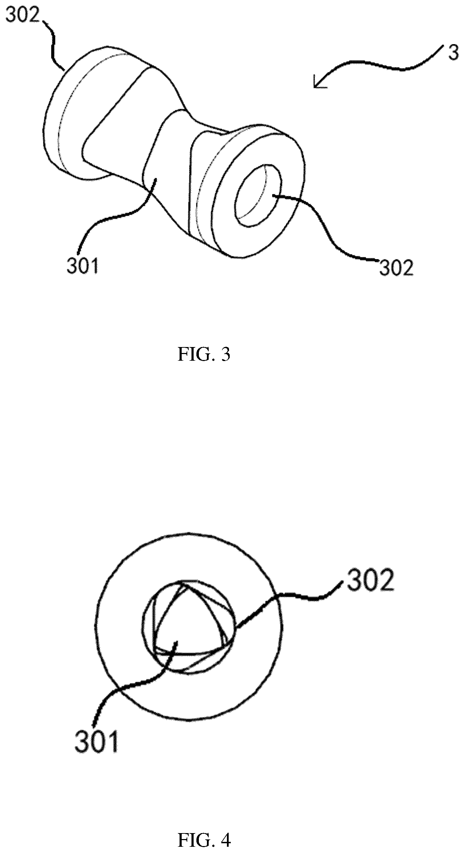

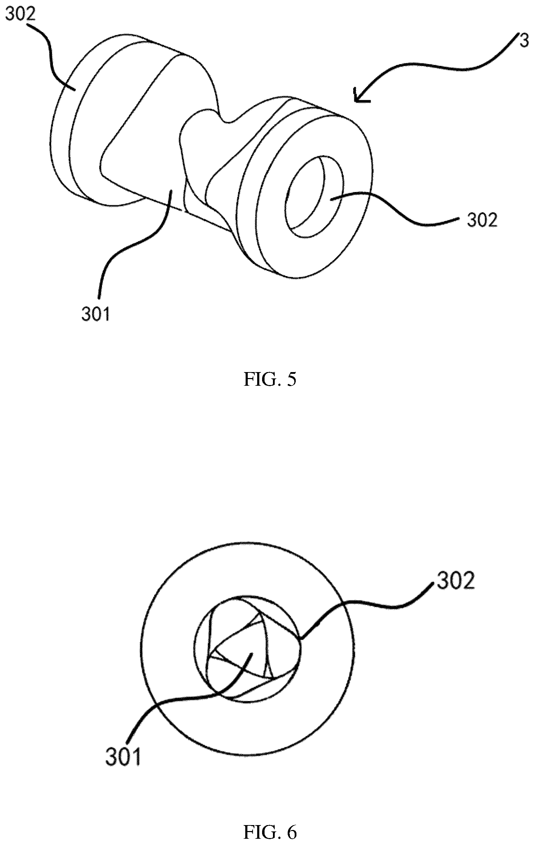

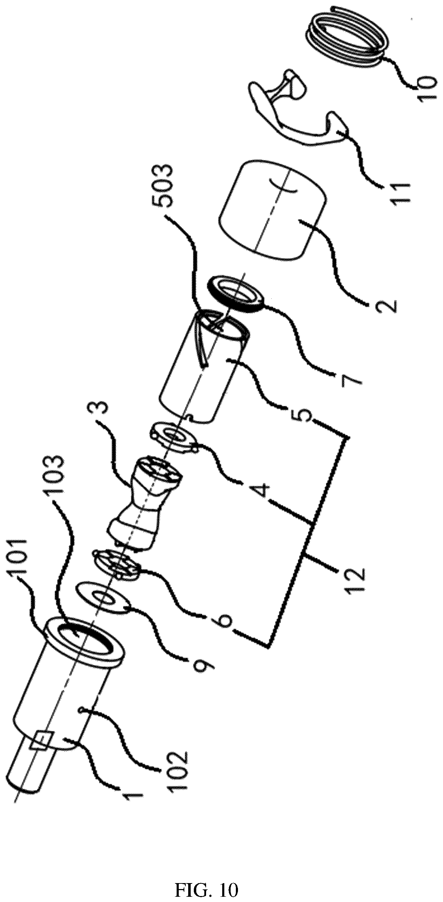

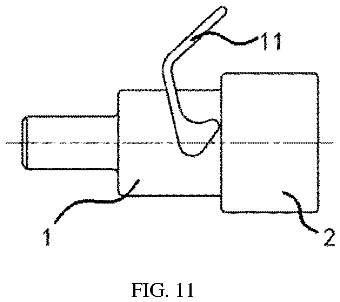

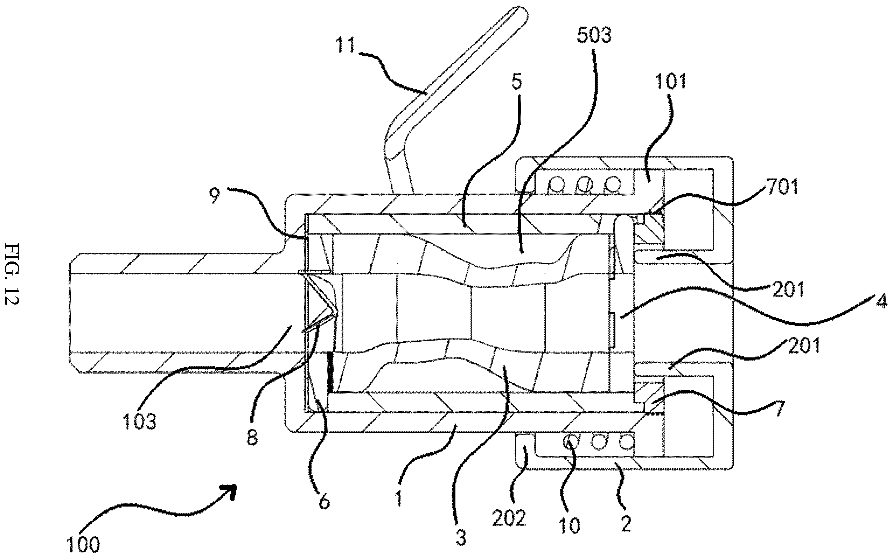

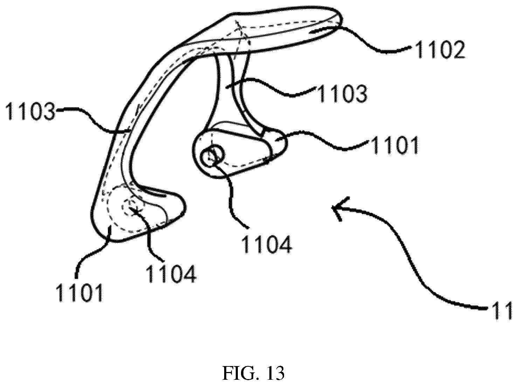

This embodiment is basically the same as the first embodiment, while the main difference is a different drive manner of moving the catheter sheath cap 2 along the axial direction of the catheter sheath housing 1. In the first embodiment, the catheter sheath housing 1 is connected in transmission with the catheter sheath cap 2 through the first thread 203. Referring to FIG. 10-FIG. 13, in this embodiment, the catheter sheath housing 1 and the catheter sheath cap 2 realize movement of the catheter sheath cap 2 along the axial direction of the catheter sheath housing 1 by cooperation between a spring 10 and a cam structure 1101. In this embodiment, the adjustable hemostasis valve device 100 further includes a spring 10 and a drive member 11. Among them, the spring 10 is sheathed on the catheter sheath housing 1, and is located between the limit ring 202 of the catheter sheath cap 2 and the limit bump 101 of the catheter sheath housing 1 after assembly; the catheter sheath housing 1 and the catheter sheath cap 2 are in gap-fit. Referring to FIG. 10, FIG. 11, and FIG. 13, the drive member 11 includes a pressing portion 1102, a connecting rod 1103, and two cam structures 1101 located at distal ends of the drive member 11, two ends of the connecting rod 1103 are respectively connected to the pressing portion 1102 and the cam structure 1101, an inner side of the cam structure 1101 forms a mounting shaft 1104, a surface of the catheter sheath housing 1 is provided with two circular holes 102, the mounting shaft 1104 of the drive member 11 is mounted in the circular holes 102, such that the drive member 11 is rotatably held on the catheter sheath housing 1, the cam structure 1101 is in contact with the distal end of the catheter sheath cap 2 and used to control displacement of the catheter sheath cap 2. The catheter sheath cap 2, under action of cooperation between the spring 10 and the cam structure 1101, can move along the axial direction of the catheter sheath housing 1. More specifically, as shown in FIG. 3, FIG. 4, FIG. 7, FIG. 8, FIG. 12, and FIG. 13, when the drive member 11 is pressed, the cam structure 1101 on the drive member 11 rotates and pushes the catheter sheath cap 2 to retreat along the axial direction of the catheter sheath housing 1 and compress the spring 10, the pushing ring 201 on the catheter sheath cap 2 disengages from the first positioning disk 4, the own elasticity of the sealing member 3 pushes the first positioning disk 4 towards the proximal end, the first positioning pins 401 on the first positioning disk 4 slide helically toward the proximal end along the sliding guide grooves 501 on the mounting cylinder 5 until contacting with the pushing ring 201 on the catheter sheath cap 2, rotate, and release the sealing member 3, thereby realizing the function of enlarging the size of the opening of the sealing member 3 in the adjustable hemostasis valve device 100. As shown in FIG. 5-FIG. 8, FIG. 12, and FIG. 13, when the drive member 11 is released, under the action of the elasticity of the spring 10, the cam structure 1101 on the drive member 11 rotates reversely, the drive member 11 resets, the elasticity of the spring 10 pushes the catheter sheath cap 2 to go ahead along the axial direction of the catheter sheath housing 1, the pushing ring 201 on the catheter sheath cap 2 pushes the first positioning disk 4, the first positioning pins 401 on the first positioning disk 4 slide helically toward the distal end along the sliding guide grooves 501 on the mounting cylinder 5, rotate, and press the silica gel sealing member 3, thereby realizing the function of reducing the size of the opening of the sealing member 3 in the adjustable hemostasis valve device 100.

It can be understood that the pushing manner of the first positioning disk 4 (i.e., the manner of driving the catheter sheath cap 2 to move along the axial direction of the catheter sheath housing 1) has multiple types, for example, spring, thread transmission, gear and rack transmission, connecting rod transmission, cam transmission, mini linear motor drive, and other manners. It is not limited by the two manners in the above embodiments.

In conclusion, the adjustable hemostasis valve device 100 of the present application includes the sealing member 3 made of silica gel or silicone rubber, the sealing member 3 includes the helical flow channel 301 located in the middle thereof axially and the straight-through flow channels 302 located at the two ends of the helical flow channel 301, and a function of adjusting the opening size of the sealing member 3 is achieved by compressing and rotating the helical flow channel 301 in the center of the sealing member 3; the outer circumference of the first positioning disk 4 is provided annularly with the plurality of first positioning pins 401, the proximal end of the mounting cylinder 5 forms the plurality of helical sliding guide grooves 501 configured for mounting the first positioning disk 4, the first positioning disk 4 can helically slide along the sliding guide grooves 501, the first positioning pins 401 cooperate with the sliding guide grooves 501 with each other, and the compressive and rotary composite motion of the sealing member 3 is realized by axially pushing and pulling the first positioning disk 4, thereby adjusting the opening size of the adjustable hemostasis valve device 100. The outer circumference of the second positioning disk 6 is provided annularly with the plurality of second positioning pins 601, the distal end of the mounting cylinder 5 form the plurality of positioning holes 502, the second positioning pins 601 of the second positioning disk 6 cooperate with the positioning holes 502 of the mounting cylinder 5 to fix the distal end of the sealing member 3 with the mounting cylinder 5. Moreover, the two ends or the middle position of the sealing member 3 is provided with the one-way valve 8 with three or more flap-leaves to further enhance the sealness of the sealing member 3, so that it can be used in the case that a pressure difference between distal and proximal ends thereof is large.

In this specification, unless otherwise clearly specified and limited, the terms "mounting", "connecting", and "connection" should be understood in the broadest sense, for example, they can be fixed connections, and can also be detachable connections, or integral connections; can be mechanical connections, and can also be electrical connections; can be direct connections, and can also be indirect connections through intermediate media; and can be an internal communication between two components. For ordinary technical personnel in this field, the specific meanings of the above terms can be understood in specific situations.

In this specification, the adjectives "first", "second", "third" and the like used to describe sequences of components are only intended to distinguish components with similar attributes, and do not mean that the described components must follow the given order or other limitations such as time, space, level, etc.

In this specification, the orientations or position relationships indicated by the terms "far", "close", "up", "down", "front", "back", "left", "right", "top", "bottom", "in", "out", "vertical", "horizontal", and so on are orientations or position relationships based on the shown in the drawings, and are only for clearness of expression of the technical solutions and convenient description, and thus should not be regarded as limit to the present invention.

In this specification, the terms "include", "comprise", or any other variation thereof are intended to encompass non-exclusive inclusion, in addition to containing those listed elements, other elements that are not explicitly listed may also be contained.

The above described are only specific embodiments of the present invention, but the protection scope of the present invention is not limited here. Any change or replacement that can be easily considered by technicians familiar with this technical field in the technical scope disclosed by the present invention should be covered in the protection scope of the present invention. Therefore, the protection scope of the present invention should be based on the protection scope of the claims.

### Industrial Applicability

The adjustable hemostasis valve device of the present invention has advantageous effects comprising:
(1) the adjustable hemostasis valve device comprises the sealing member made of silica gel or silicone rubber, which comprises the helical flow channel located in the middle of the sealing member axially and straight-through flow channels located at the two ends of the helical flow channel, and a function of adjusting the opening size of the sealing member is achieved by compressing and rotating the helical flow channel in the center of the sealing member;
(2) the sealing member is provided with the one-way valve in a shape of three or more flap-leaves to further enhance its sealness, so that it can be used in the case that a pressure difference between distal and proximal ends thereof is large;
(3) the first positioning disk, the second positioning disk, and the mounting cylinder form the opening adjustment mechanism, the first positioning disk and the second positioning disk are provided with the first positioning pins and the second positioning pins, the mounting cylinder is provided with the helical sliding guide groove, the compressive and rotary composite motion of the sealing member is realized by axially pushing and pulling the first positioning disk, thereby adjusting the opening size of the sealing member in the adjustable hemostasis valve device;
(4) the movable catheter sheath cap with a thread structure can accurately control the axial movement of the catheter sheath cap by thread rotation so as to push the first positioning disk, thereby realizing accurate control of the opening size of the sealing member in the adjustable hemostasis valve device;
(5) the axial movement of the catheter sheath cap along the catheter sheath housing is realized through the cooperation of the spring and the cam structure of the drive member, the opening size of the sealing member in the adjustable hemostasis valve device is controlled through the drive member for passage of instruments, the spring automatically resets after the drive member is released to drive the catheter sheath cap to move axially to maintain sealness, and the drive member and the spring cooperate with each other so as to realize adaptive adjustment for instruments of different diameters.

## Claims

1. An adjustable hemostasis valve device (100) comprising a catheter sheath and a sealing member (3), the catheter sheath is internally hollow, the sealing member (3) is located within the catheter sheath, the sealing member (3) is an elastic member comprising a helical flow channel (301) extending axially, a proximal end and a distal end of the catheter sheath is movable relative to each other axially, and the proximal end of the catheter sheath, when moving axially, is capable of compressing and rotating, or releasing and rotating, the sealing member (3) to adjust the size of an opening of the helical flow channel (301) of the sealing member (3) to accommodate different standards of surgical instruments passing through the helical flow channel (301).

2. The adjustable hemostasis valve device (100) according to claim 1, wherein the catheter sheath comprises a catheter sheath housing (1) located at a distal end and a catheter sheath cap (2) located at a proximal end, the catheter sheath housing (1) and the catheter sheath cap (2) are movable relative to each other axially; a distal end portion of the catheter sheath cap (2) extends inwardly to form a limit ring (202), a position of the catheter sheath housing (1) close to the proximal end is provided annularly with a limit bump (101), and the limit ring (202) of the catheter sheath cap (2) and the limit bump (101) of the catheter sheath housing (1) cooperate with each other to limit the relative displacement stroke between the catheter sheath cap (2) and the catheter sheath housing (1).

3. The adjustable hemostasis valve device (100) according to claim 2, further comprising an opening adjustment mechanism (12), wherein the opening adjustment mechanism (12) comprises a first positioning disk (4) and a mounting cylinder (5), the first positioning disk (4) is fixedly connected to a proximal end of the sealing member (3), the sealing member (3) is mounted in the mounting cylinder (5) after being fixedly connected to the first positioning disk (4), a plurality of first positioning pins (401) are distributed around an outer periphery of the first positioning disk (4), and a plurality of helical sliding guide grooves (501) matching the positioning pins (401) are formed at a proximal end of the mounting cylinder (5); when the catheter sheath cap (2) moves axially, the first positioning pins (401) of the first positioning disk (4) slide along the sliding guide grooves (501) of the mounting cylinder (5) to adjust the size of the opening of the helical flow channel (301) of the sealing member (3).

4. The adjustable hemostasis valve device (100) according to claim 3, wherein the opening adjustment mechanism (12) further comprises a second positioning disk (6), the second positioning disk (6) is fixedly connected to a distal end of the sealing member (3), a plurality of second positioning pins (601) are distributed around an outer periphery of the second positioning disk (6), and a plurality of positioning holes (502) are formed at a distal end of the mounting cylinder (5); the second positioning pins (601) of the second positioning disk (6) cooperate with the positioning holes (502) of the mounting cylinder (5) to fix a distal end of the sealing member (3) with the mounting cylinder (5).

5. The adjustable hemostasis valve device (100) according to claim 4, wherein surfaces of both the first positioning disk (4) and the second positioning disk (6) close to the sealing member (3) are provided with first steps (402), corresponding positions of the two ends of the sealing member (3) are provided with second steps (303), the first steps (402) and the second steps (303) are staggered along a circumferential direction, and the first positioning disk (4) and the second positioning disk (6) are bonded together with the sealing member (3) through the first steps (402) and the second steps (303).

6. The adjustable hemostasis valve device (100) according to claim 5, further comprising a one-way valve (8), wherein the one-way valve (8) is provided axially at two ends or in the middle of the sealing member (3).

7. The adjustable hemostasis valve device (100) according to claim 3, wherein a push ring (201) is formed on an inner side of the catheter sheath cap (2), a distal end of the push ring (201) is in contact with a proximal end of the first positioning disk (4), and the push ring (201) is coupled with the first positioning disk (4) when the catheter sheath cap (2) moves axially.

8. The adjustable hemostasis valve device (100) according to claim 3, further comprising a fixing ring (7), wherein the fixing ring (7) is fixedly connected to the proximal end of the mounting cylinder (5), and an outer periphery of the fixing ring (7) is connected by thread with an inner wall of the proximal end of the catheter sheath housing (1) to fix the mounting cylinder (5) within the catheter sheath housing (1).

9. The adjustable hemostasis valve device (100) according to claim 2, wherein the catheter sheath cap (2) is connected by thread with the proximal end of the catheter sheath housing (1), and by rotating the catheter sheath cap (2), it is capable of being controlled to move along an axial direction of the catheter sheath housing (1).

10. The adjustable hemostasis valve device (100) according to any one of claims 2-8, further comprising a spring (10) and a drive member (11), wherein the spring (10) is sheathed on the catheter sheath housing (1) and located between the limit ring (202) of the catheter sheath cap (2) and the limit bump (101) of the catheter sheath housing (1), and the catheter sheath housing (1) and the catheter sheath cap (2) are in gap-fit; the drive member (11) is rotatably fixed to the catheter sheath housing (1), a distal end of the drive member (11) has a cam structure (1101), the cam structure (1101) is in contact with the distal end of the catheter sheath cap (2) for controlling displacement of the catheter sheath cap (2), and the catheter sheath cap (2) is movable axially along the catheter sheath housing (1) under the cooperative action of the spring (10) and the cam structure (1101).

11. The adjustable hemostasis valve device (100) according to claim 10, wherein the drive member (11) comprises a pressing portion (1102) and the cam structure (1101) formed by extension from two sides of the pressing portion (1102), the cam structure (1101) is provided thereon with a mounting shaft (1104), the drive member (11) is rotatably secured on the catheter sheath housing (1) through the mounting shaft (1104).
